# FASCICULE DE BREVET EUROPEEN

(11) **EP 1 420 612 B1**
(45) Date de publication et mention de la délivrance du brevet: **16.04.2008**
(21) Numéro de dépôt: 03292730.3
(22) Date de dépôt: 31.10.2003
(51) Int. Cl.: H05B 3/62, C03B 23/043, B01L 7/00, A61L 2/04

(54) **Brûleur permettant le travail de matériau à haute température**
Brenner für auf hoher Temperatur zu behandelndes Material
Burner able to treat high temperature material

(30) Priorité: 14.11.2002 FR 0214248
(43) Date de publication de la demande: 19.05.2004
(73) Titulaire: Chemla, Richard, 91390 Morsang sur Orge (FR)
(72) Inventeur: Chemla, Richard, 91390 Morsang sur Orge (FR)
(74) Mandataire: Wagret, Frédéric

(56) Documents cités:
- EP-A- 0 826 420
- US-A- 3 168 640
- US-A- 5 112 376

## Description

Le dispositif selon l'invention concerne un brûleur permettant le chauffage d'un élément et/ou le travail à haute température d'un matériau de type verre et/ou la stérilisation du milieu environnant.

Dans des laboratoires, de recherche par exemple, il est nécessaire pour effectuer des manipulations précises dans des milieux de culture, d'utiliser des pipettes Pasteur ou des râteaux de verre qui ont été formées à partir de baguettes de verre creuses ou pleines, disponibles dans le commerce.

Selon l'art antérieur, il est connu de travailler ces baguettes de verre, c'est-à-dire de les chauffer et de les étirer, à partir d'un brûleur à gaz.

En effet, le verre ne devient malléable qu'à partir de très hautes températures. Ainsi, il se ramollit vers 800°C et ne peut être travaillé que vers 900°C.

Les dispositifs connus fonctionnent à partir d'une source d'énergie gazeuse, soit des cartouches de gaz, soit une alimentation de gaz de ville.

Cependant, ce type de dispositif n'est pas satisfaisant puisque l'énergie gazeuse n'est pas la plus facilement accessible et elle est considérée comme étant dangereuse de par les fuites qui peuvent intervenir et le risque d'explosion.

De plus, ces dispositifs se présentent sous la forme d'une flamme nue, ce qui cause d'autres problèmes de sécurité et un travail du verre assez long.

Il est connu selon EP-A-0 826 420 de réaliser un dispositif de chauffage électrique pour verrerie de laboratoire sous la forme d'un bras articulé et à l'aide de résistances électriques.

Toutefois, ce dispositif ne permet pas de réaliser un travail du verre, notamment à partir de baguettes de verre creuses ou pleines de manière rapide et sûre.

Le dispositif décrit dans US 3 168 640 ne permet également pas le travail du verre à des températures élevées d'une manière rapide et sûre.

Le brevet américain n°5112376 divulgue un procédé et un appareil pour la calibration des sections des tubes de verre et utilise des résistances électriques pour travailler le diamètre du verre des tubes. Néanmoins, l'appareil ne présente pas un accès facile et des conditions de sécurité optimales.

Le dispositif selon l'invention résout ce problème en proposant un dispositif permettant le travail du verre dans des temps raisonnables et dont le chauffage est réalisé grâce à des moyens plus surs et plus facilement accessibles.

Le dispositif selon l'invention résout ce problème en proposant un dispositif permettant le travail du verre dans des temps raisonnables et dont le chauffage est réalisé grâce à des moyens plus surs et plus facilement accessibles.

Afin d'améliorer les conditions de sécurité lors du chauffage d'un élément et/ou le travail à haute température d'un matériau de type verre et/ou la stérilisation du milieu environnant, le dispositif selon l'invention est un brûleur apte à réaliser à la fois le chauffage d'un élément et la travail à haute température, ledit chauffage et ledit travail étant réalisés à partir de résistances électriques.

De manière complémentaire, la brûleur est également apte à réaliser la stérilisation du milieu environnant à partir de résistances électriques.

Pour obtenir un confinement de la chaleur et ainsi atteindre des températures élevées, le brûleur comporte une zone de chauffage dite zone de four apte à permettre le travail du matériau de type verre, la zone de four étant en forme de U selon une vue en section droite, de telle manière que la branche supérieure et la branche inférieure du U soient horizontales.

Afin d'obtenir une température suffisante pour permettre le travail du verre, les parois du four sont réalisées à partir d'au moins deux zones résistives électriques formant deux des trois parois de ladite zone de four.

Avantageusement, deux des trois parois de la zone de four sont composées par une première zone résistive dite zone résistive supérieure correspondant à la branche supérieure du U, et par une deuxième zone résistive, dite zone résistive inférieure, correspondant à la branche inférieure du U.

De manière encore plus avantageuse, ladite zone de four est délimitée par au moins trois zones résistives électriques formant lesdites trois parois, la troisième zone résistive chauffante dite zone résistive de fond correspondant à la base dudit U

De manière à pouvoir travailler le verre dans des temps raisonnables, la température dans la zone de four est au maximum comprise entre 850°C et 950°C, et atteint de manière préférentielle 900°C.

Afin d'obtenir un courant de convection d'air approprié, le brûleur comporte des moyens d'acheminement d'air définissant un volume intérieur délimité par trois parois en matériau réfractaire, et de manière préférentielle en vermiculite.

De manière avantageuse, le brûleur comporte une zone d'aspiration d'air, située au niveau de la liaison entre des moyens de maintien au sol, sur lesquels reposent les dites parois en matériau réfractaire, la zone d'aspiration d'air et les moyens d'acheminement d'air comportant une zone résistive dite zone résistive de base.

Afin d'obtenir un courant de convection d'air approprié, les moyens d'acheminement d'air sont composés de la zone d'aspiration d'air, suivie d'une zone verticale de préchauffage de l'air formant cheminée vers la zone de four, la zone de préchauffage de l'air comportant un élément résistif vertical sensiblement centré, dit résistance de préchauffage.

De manière avantageuse, la zone de préchauffage présente une zone résistive verticale, dite zone résistive de parois de préchauffage correspondant à une des parois de la zone de préchauffage.

Afin de permettre le chauffage d'un élément, le brûleur comporte une zone de chauffage ouverte située au-dessus de la zone résistive supérieure du four au niveau de laquelle la température est de l'ordre de 800°C.

Afin de protéger l'utilisateur d'un éventuel contact avec les éléments chauffants, le brûleur comporte un boîtier incluant les moyens d'acheminement d'air, les moyens de maintien au sol ainsi que les différentes zones résistives.

Afin d'optimiser la valeur de la température obtenues dans la zone de four, un dissipateur métallique formé par trois flancs en U est disposé entre lesdits moyens d'acheminement d'air et les zones résistives.

Afin de ne pas bloquer l'accès à la zone de four, le dissipateur présente sur ses deux flancs parallèles une découpe en forme de U correspondant à la zone de four.

Avantageusement, la distance entre le dissipateur et les parois des moyens d'acheminement de l'air est comprise entre 3 et 8 mm, et de manière préférentielle entre 4 et 6 mm, et de manière encore plus préférentielle 5 mm.

Afin d'éviter un contact direct de l'utilisateur et/ou du matériau à travailler et/ ou de l'élément à chauffer avec les différentes zones résistives, la brûle comporte un élément de protection, conformé en S majuscule selon une vue latérale, apte à s'emboîter dans le dissipateur

La présente invention sera maintenant bien mieux comprise à la lumière de la description suivante, qui ne limite aucunement l'invention en référence aux dessins annexés dans lesquels :
- La figure 1 représente en perspective latérale le boîtier métallique qui recouvre le dispositif de chauffage ;
- La figure 2 représente une vue en coupe latérale du dispositif selon l'invention ;
- La figure 3 représente une vue en perspective plongeantes des résistances électriques, et de l'enceinte en bloc isolant ;
- La figure 4 représente également une vue en perspective des éléments de la figure 3;
- La figure 5 représente le dissipateur en perspective frontale ;
- La figure 6 représente une vue latérale du dissipateur ;
- La figure 7 représente en perspective l'élément de protection ;
- La figure 8 représente une vue en perspective du dissipateur associé à l'élément de protection.

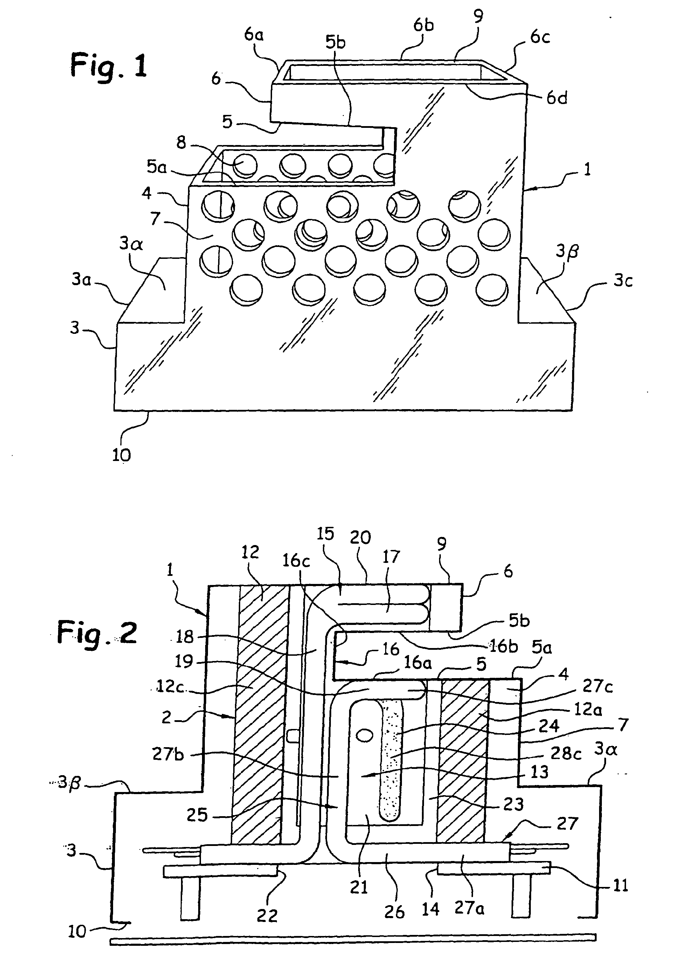

Sur la figure 1 est représenté un boîtier 1 ou plus précisément un étui dans lequel doit être contenu le dispositif chauffant 2 (non représenté sur la figure 1).

Par mesure de clarté et de commodité, dans la description le dispositif selon l'invention est appelé brûleur

Le boîtier 1 est composé d'une embase 3 parallélépipédique et d'une enceinte parallélépipédique 4.

L'enceinte 4 présente une hauteur supérieure à celle de l'embase 3. Cette dernière présente deux décrochements 3α et 3β du fait que l'enceinte 4 présente une longueur, suivant une dimension, inférieure à la longueur correspondante de l'enceinte 4.

L'embase 3 comprend quatre côtés égaux et parallèles deux à deux, respectivement (3a, 3c) et (3b, 3d).

L'enceinte 4 comprend, sur deux de ses côtés parallèles, une découpe 5 en forme de U formant une partie supérieure à décrochement 6 comprenant des côtés, égaux et parallèles deux à deux, (6a, 6c) et (6b, 6d).

Le U de la découpe 5 est horizontal et présente une branche inférieure 5a et une branche supérieure 5b ; cette dernière est plus longue que la branche inférieure 5b.

La partie inférieure de l'enceinte 4, située sous la partie à décrochement 6, dite partie à lumières 7, comporte des côtés égaux et parallèles deux à deux, (7a, 7c) et (7b, 7d) et est pourvue de lumières 8.

La partie à décrochement 6 présente, selon la vue latérale de la figure 1, une longueur suivant l'axe de la découpe 5 en U, inférieure à la partie inférieure ajourée 5 de l'enceinte 4.

Le boîtier 1 est ouvert sur ses faces supérieure 9 et inférieure 10, ce qui permet d'aspirer l'air situé sous l'embase 3 au niveau de la face inférieure 10, pour ensuite le réchauffer grâce au dispositif chauffant 2 (non représente sur la figure 1), qui finalement évacue cet air au niveau de la face supérieure 9.

Une convection de chaleur est donc créée quand le dispositif chauffant 2 est disposé dans le boîtier 1.

Le boîtier 1 permet de protéger l'utilisateur d'éventuelles brûlures lors de l'emploi du brûleur.

La figure 2 représente une vue en coupe du brûleur composé du boîtier 1 avec le dispositif chauffant 2.

Le dispositif chauffant 2 est composé de moyens de maintien au sol 11 (par exemple sous la forme de quatre pieds) ainsi que de moyens d'isolation 12, constitués d'un ensemble parallélépipédique composé de quatre parois (12a, 12b, 12c, 12d) en matériau isolant thermiquement, de préférence réfractaire, tel que de la vermiculite.

Une fois le dispositif chauffant 2 installé dans le boîtier 1, les parois réfractaires (12a, 12b, 12c, 12d) sont conformées de manière telles qu'elles permettent un accès libre à la découpe 5 en forme de U prévue sur l'enceinte 4 du boîtier 1.

La paroi frontale 12a est la paroi la plus proche de l'utilisateur quand celui-ci utilise le brûleur, la paroi dorsale 12c est la paroi la plus éloignée de l'utilisateur quand celui-ci utilise la brûleur, les parois 12b et 12d sont les parois latérales du dispositif chauffant.

Les parois frontale 12a et dorsale 12c sont de hauteurs différentes, la paroi 12a ne dépassant pas le plan horizontal défini par la branche inférieure 5b, la parois 12c ne dépassant pas le plan horizontal défini par la surface 9.

Les parois latérales 12b et 12d (non représentées sur la figure 2) sont toutes deux identiques et présentent une première partie ayant la hauteur de la paroi frontale 12a et une seconde partie ayant la hauteur de la paroi dorsale 12c.

Les moyens d'isolation 12, et plus particulièrement les parois réfractaires (12a, 12b, 12c, 12d) définissent un volume intérieur 13 de forme générale parallélépipédique.

Le volume intérieur 13 est ouvert sur ses deux faces, la face supérieure étant confondue avec la face 9 du boîtier 1 et une face inférieure 14 au niveau de la liaison entre les moyens de maintien au sol 11 et les parois réfractaires (12a, 12b, 12c, 12d).

Dans le volume intérieur 13 des parois réfractaires (12a, 12b, 12c, 12d) est disposé un ensemble de résistances électriques 15.

Selon la figure 2, l'ensemble résistif 15 est composé de quatre résistances filaires entremêlées les unes avec les autres et définissant ainsi des zones résistives, chacune d'elle étant définie par au moins une résistance filaire.

L'ensemble résistif 15 définit tout d'abord une zone de four 16.

La zone de four 16 est en forme de U selon une vue en section droite, de telle manière que les branches inférieure 16a et supérieure 16b du U soient horizontales.

Les branches inférieure 5a et supérieure 5b du boîtier 1 sont sensiblement parallèles et/ ou confondues avec les branches inférieure 16a et supérieure 16b de la zone de four 16.

Les banches inférieure 16a et supérieure 16b de ladite zone de four 16 définissent deux des trois parois de la zone de four 16.

La troisième paroi de la zone de four 16 est la paroi de fond 16c verticale et correspondant au fond du U.

Chacune de ces trois parois (16a, 16b, 16c) définit une zone résistive électrique chauffante.

La branche supérieure 16b définit une première zone résistive dite zone résistive supérieure 17.

La paroi de fond 16c définit une seconde zone résistive dite zone résistive de fond 18.

La branche inférieure 16a définit une troisième zone résistive dite zone résistive inférieure 19.

Au-dessus de la zone résistive supérieure 17, et plus précisément au niveau de la surface 9, est située une zone de chauffage ouverte 20.

Le volume intérieur 13 comporte également des moyens d'acheminement d'air 21 composée, à sa base d'une zone d'aspiration d'air 22, suivie d'une zone verticale de préchauffage d'air 23.

La zone d'aspiration d'air 22 est confondue avec l'embouchure basse 14 comme ci-dessus décrite.

La zone de préchauffage 23 comporte un élément résistif filaire vertical centré dit résistance de préchauffage 24.

Les parois de la zone de préchauffage d'air 24 sont définies par la paroi réfractaire frontale 12a et par une autre paroi verticale, parallèle à la paroi frontale 12a, définie par une zone résistive dite zone résistive de parois de préchauffage 25.

La zone d'aspiration d'air 22 comporte une zone résistive 26 dite zone résistive de base.

L'ensemble résistif 15 comporte quatre résistances filaires, dont la résistance de préchauffage 24.

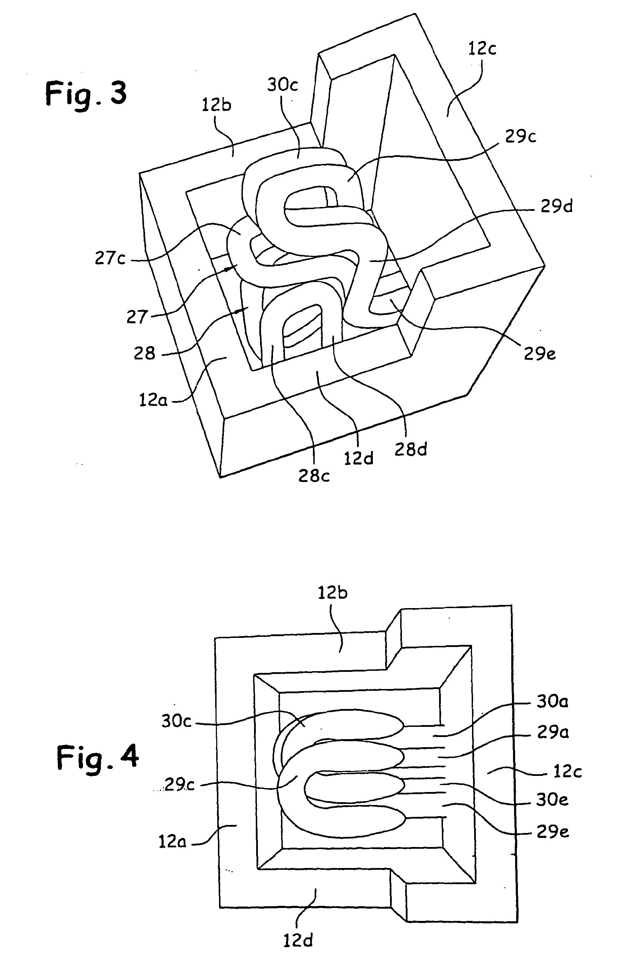

La résistance frontale 27 filaire comporte :
- une première partie horizontale 27a comprise dans la zone résistive de base 26 ;
- une seconde partie résistive verticale 27b comprise dans la zone résistive de parois de chauffage 24 ;
- une troisième partie horizontale 27c comprise dans la zone résistive inférieure 19 et sous la forme d'une boucle (voir figures 3 et 4), permettant ainsi de couvrir une surface de chauffage plus importante ;
- une quatrième partie 27d verticale, parallèle à la deuxième partie 27b, comprise également dans la zone résistive de parois de chauffage 24, et
- une cinquième partie 27e horizontale, parallèle à la première partie 27a, comprise également dans la zone résistive de base 26.

L'élément résistif 15 comporte également une deuxième résistance filaire 28 dite résistance frontale centrée qui est composée d' :
- une première partie 28a horizontale comprise dans la zone résistive de base 26 ;
- une seconde partie 28b verticale formant une des parois de la zone de préchauffage 23 et comprise dans la zone résistive de parois de préchauffage 25, de longueur approximative la hauteur de la paroi frontale 12a ;
- une troisième partie 28c verticale centrée dans la zone de préchauffage 23 ou résistance de préchauffage 24 ;
- une quatrième partie 28d verticale, parallèle à la seconde partie verticale 28b de la résistance frontale centrée 28 formant une des parois de la zone de préchauffage 23 et comprise dans la zone résistive de parois de préchauffage 25, de longueur approximative la hauteur de la paroi frontale 12a , et
- une cinquième partie 28e horizontale, parallèle à la première partie horizontale 28a de la résistance frontale centrée 28 comprise dans la zone résistive de base 26.

Les résistances frontale 27 et frontale centrée 28 sont légèrement décalées l'une par rapport à l'autre dans un plan perpendiculaire au plan du sol.

L'élément résistif 15 comporte une troisième résistance filaire dite résistance dorsale supérieure 29 qui est composée d' :
- une première partie 29a horizontale au niveau de la zone d'aspiration d'air 22 comprise dans la zone résistive de base 26 ;
- une deuxième partie 29b verticale comprise entre la paroi dorsale en réfractaire 12c et la zone résistive de parois de préchauffage 25, de longueur approximativement la hauteur de la paroi dorsale 12d, et dont une partie est comprise dans la zone résistive de fond 18 ;
- une troisième partie 29c correspondant à la zone de chauffage ouverte 20 sous la forme d'une boucle (voir figures 3 et 4) ;
- une quatrième partie 29d, parallèle à la deuxième partie 29b, comprise entre la paroi dorsale en réfractaire 12c et la zone résistive de parois de préchauffage 25, de longueur approximativement la hauteur de la paroi dorsale 12d, et dont une partie est comprise dans la zone résistive de fond 18, et
- une cinquième partie 29e, parallèle à la première partie 29a, horizontale au niveau de la zone d'aspiration d'air 22 comprise dans la zone résistive de base 26 .

L'élément résistif 15 comporte une quatrième résistance filaire dite résistance dorsale inférieure 30 qui est composée d' :
- une première partie 30a horizontale au niveau de la zone d'aspiration d'air 22 comprise dans la zone résistive de base 26, décalé d'environ d'un diamètre de la résistance frontale supérieure 29 ;
- une deuxième partie 30b verticale comprise entre la paroi dorsale en réfractaire 12d et la zone résistive de parois de préchauffage 25, de longueur approximativement la hauteur de la paroi dorsale 12c, et dont une partie est comprise dans la zone résistive de fond 18 ;
- une troisième partie 30c correspondant à la zone résistive supérieure 17 sous la forme d'une boucle (voir figures 3 et 4) ;
- une quatrième partie 30d, parallèle à la deuxième partie 30b, comprise entre la paroi dorsale en réfractaire 12d et la zone résistive de parois de préchauffage 25, de longueur approximativement la hauteur de la paroi dorsale 12c et dont une partie est comprise dans la zone résistive de fond 18, et
- une cinquième partie 30e, parallèle à la première partie 30a, horizontale au niveau de la zone d'aspiration d'air 22 comprise dans la zone résistive de base 26 .

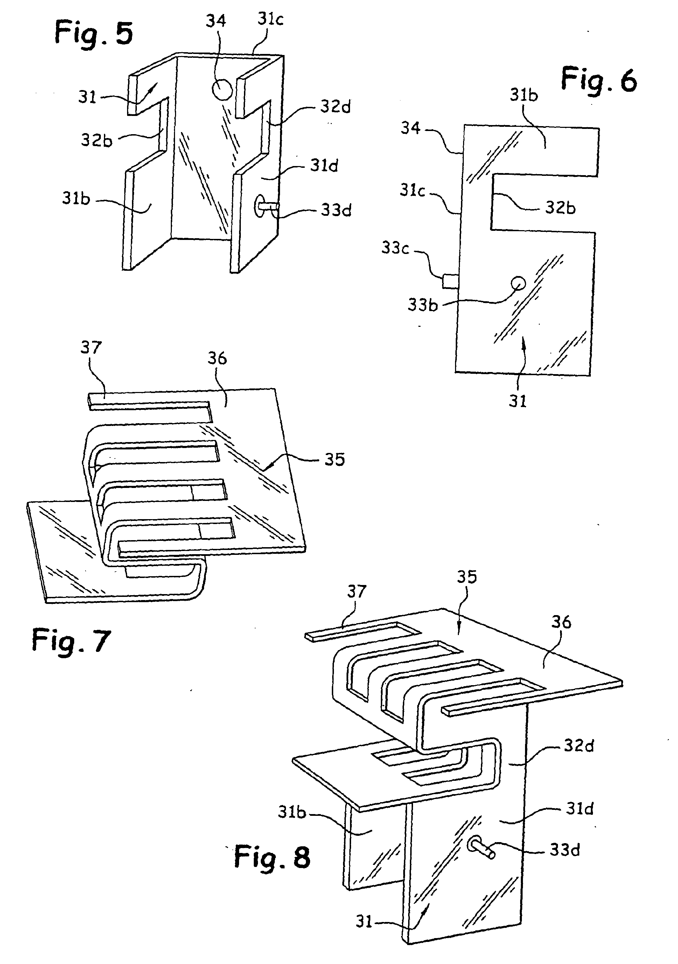

Une fois le dispositif chauffant 2 emboîté dans le boîtier 1, un dissipateur 31 (voir figure 5) est inséré dans le volume intérieur 13 dans l'espace délimité par les parois réfractaires (12a, 12b, 12c, 12d) et les quatre résistances filaires (24, 28, 29, 30).

Le dissipateur 31 est un élément métallique composé de deux flancs latéraux 31 b et 31 d et d'un flanc dorsal 31 c.

La flanc dorsal 31 c est de forme quadrangulaire de longueur légèrement inférieure à la hauteur de la paroi dorsale 12c.

Les flancs latéraux 31 b et 31d ont les mêmes dimensions et présentent tous deux une ouverture en forme de U (32b, 32d).

Le dissipateur 31 est conformé de manière à ce que les ouvertures en forme de U (32b, 32d) des flancs latéraux (31b, 31d) coïncident avec les ouvertures en forme de U de la zone de four 16 et l'ouverture 8 en forme de U du boîtier 1.

Le dissipateur 31 comporte sur la surface extérieure de chacun de ses flancs (31 b, 31 c, 31d) un élément cylindrique (respectivement 33b, 33c, 33d).

Les éléments cylindriques (33b, 33c, 33d) permettent au dissipateur d'être maintenu en place dans le volume intérieur 13 du dispositif chauffant 2.

Les éléments cylindriques (33b, 33c, 33d) présentent idéalement une hauteur comprise entre 3 et 8 mm, de manière préférentielle entre 4 et 6mm, et de manière encore plus préférentielle 5mm.

Le dissipateur 31 présente avantageusement sur son flanc dorsal 31d une lumière de forme circulaire 34.

Ainsi, la distance entre le dissipateur 31 et les parois des moyens d'acheminement de l'air (12b, 12c, 12d) est comprise entre 3 et 8 mm, et de manière préférentielle entre 4 et 6 mm, et de manière encore plus préférentielle 5 mm

Finalement, un élément de protection 35 (voir figure 7), conformée en S, s'emboîte dans le dissipateur 31 (voir figure 8).

L'élément de protection 35 présente sur sa partie supérieure une grille 36 présentant des lumières ou des découpes 37, représentées sous une forme quadrangulaire sur les figures 7 et 8.

Les lumières 37 peuvent être de tout type connu, par exemple circulaires, trapézoïdales,...

La grille 36 permet de recouvrir la surface supérieure 9 du boîtier 1 et ainsi d'éviter un contact direct de l'élément à chauffer ou de l'utilisateur avec les résistances chauffantes (29, 30).

Un tel contact est également évité au niveau de la zone de four 16.

Une fois, l'élément de protection 35 insérée dans le dissipateur 31 qui est lui même disposé dans le volume intérieur 13, il est alors possible, par exemple de chauffer un élément au niveau de la zone de chauffage ouverte 20, en plaçant l'élément à chauffer sur la grille 36.

Le brûleur constitue, grâce à ses différentes zones résistives (17, 18, 19, 25, 26), un dispositif fonctionnant à l'aide de moyens de convection d'air.

En effet, les différentes zones résisitves permettent la création de masses d'air à des températures différentes interagissant entre elles, d'où la convection.

Les échanges d'air sont réalisés entre l'air froid aux alentours du brûleur et l'air chauffé dans le brûleur.

Ainsi, l'air froid situé sous la zone résistive de base 26 est aspiré dans le volume intérieur 13 où il est ensuite préchauffé au niveau de la zone de préchauffage 23.

De plus, de nombreux courants de convection entre le dissipateur 31 et les parois en matériau réfractaire (12a, 12b, 12c, 12d) ont également lieu.

La lumière de forme circulaire 34, située sur le flanc dorsal 31c, permet également la convection d'air, c'est-à-dire un échange d'air chaud et d'air frais entre les zones chauffantes et le milieu extérieur.

Il en est de même pour les lumières 8 situées sur le boîtier 1.

En cours de fonctionnement, la température dans la zone de four varie entre 850°C et 950°C, et avoisine de manière préférentielle 900°C.

La température dans la zone de chauffage ouverte 20 avoisine au maximum les 800°C.

Ce niveau de température dans la zone de four 16 permet le travail de matériau de type verre.

Ainsi, des baguettes en verre creuses ou pleines peuvent être introduites dans la zone de four, l'utilisateur tenant les extrémités de la baguette en verre.

L'utilisateur peut ainsi fabriquer des pipettes Pasteur, des râteaux de verre ou des coudes, d'une manière rapide.

Le brûleur est relié électriquement sur secteur selon tout moyen connu par l'homme de l'art.

Il peut être également prévu des moyens de réglage de la température de type connu pour permettre l'obtention d'une température désirée au niveau de la zone de four 16 et/ ou de la zone de chauffage ouverte 20.

Le brûleur peut également être utilisé pour stériliser le milieu environnant dans lequel il est placé.

En effet, le dégagement de chaleur entraîné par le brûleur permet la création d'un courant de convection d'air circulant du brûleur vers l'extérieur.

Ainsi, l'air avoisinant est chassé et le milieu environnant est ainsi stérilisé.

Ce mode de fonctionnement peut être ainsi avantageusement utile quand on cherche, par exemple, à stériliser des boîtes de Pétri situées sur une paillasse de laboratoire autour du brûleur pour pouvoir ainsi effectuer des manipulations en milieu stérile sur ces boîtes.

## Revendications

1. Brûleur permettant le chauffage et le travail à haute température d'un matériau du type verre, comportant des résistances électriques (24, 27, 28, 29, 30), un dispositif chauffant (2) formé de parois (12a, 12b, 12c, 12d), un boîtier (1) dans lequel s'emboîte le dispositif chauffant (2), et une zone de four (16) apte au travail à haute température du dit matériau, **caractérisé en ce que** :
la zone de four (16) présente une forme de U selon une vue en section droite, de telle manière que la branche supérieure (16b) et la branche inférieure (16a) soient horizontales.

2. Brûleur selon la revendication précédente, **caractérisé en ce qu'**il comporte une zone de chauffage ouverte du dit matériau (20) distincte de la zone de four (16).

3. Brûleur selon l'une des revendications précédentes, **caractérisé en ce qu'**il comporte un dissipateur (31) apte à être inséré dans un volume (13) délimité par les parois (12a, 12b, 12c, 12d) et les quatre résistances électriques (24, 28, 29, 30).

4. Brûleur selon la revendication 3, **caractérisé en ce que** des lumières (8, 34) situés sur le boitier (1) et sur le flanc dorsal (31 c) du dissipateur (31) permettent la convection d'air.

5. Brûleur selon l'une des revendications 3 à 4, **caractérisé en ce qu'**il comporte une première zone résistive dite zone résistive supérieure (17) correspondant à ladite branche supérieure (16b) dudit U, et une deuxième zone résistive dite zone résistive inférieure (19) correspondant à ladite branche inférieure (16a) dudit U.

6. Brûleur selon la revendication 5, **caractérisé en ce que** la zone de chauffage ouverte (20) est située au-dessus de la zone résistive supérieure (17) du four.

7. Brûleur selon l'une des revendications 3 à 6, **caractérisé en ce que** le dissipateur métallique (31) est formé par trois flancs (31 b, 31 c, 31 d) qui constituent un U.

8. Brûleur selon l'une des revendications 3 à 7, **caractérisé en ce que** ledit dissipateur (31) présente sur ses flancs parallèles (31 b, 31 d) une découpe (32b, 32d) en forme de U.

9. Brûleur selon l'une des revendications 3 à 8, **caractérisé en ce qu'**il comporte un élément de protection (35) apte à s'emboîter dans ledit dissipateur (31) permettant d'éviter un contact direct des dites résistances électriques (17, 18, 19, 25, 26) avec ledit matériau à travaille ou ledit élément à chauffer.

10. Brûleur selon l'une des revendications 3 à 9, **caractérisé en ce que** le dissipateur (31) est conformé de manière à ce que les ouvertures en forme de U (32b, 32d) des flancs latéraux (31b, 31d) coïncident avec les ouvertures en forme de U (16a, 16b) de la zone de four (16) et une ouverture en forme de U (5a, 5b) du boîtier (1).

11. Brûleur selon l'une des revendications précédentes, **caractérisé en ce qu'**il comporte au moins deux zones résistives électriques (17, 18, 19, 25) sur deux parois (16a, 16b, 16c) de la dite zone de four (16).

12. Brûleur selon l'une des revendications précédentes, **caractérisé en ce que** ladite zone de four (16) est délimitée par trois zones résistives électriques (17, 18, 19) formant les trois parois (16a, 16b, 16c) de la dite zone de four (16).

13. Brûleur selon l'une des revendications précédentes, **caractérisé en ce qu'**il comporte des moyens d'acheminement d'air (21) définissant le volume intérieur (13) délimité par les quatre parois en matériau réfractaire (12a, 12b, 12c, 12d) du dispositif chauffant (2).

14. Brûleur selon la revendication 13, **caractérisé en ce qu'**il comporte une zone d'aspiration d'air (22), située au niveau de la liaison entre des moyens de maintien au sol (11), sur lesquels reposent les parois en matériau réfractaire (12a, 12b, 12c, 12d), ladite zone d'aspiration d'air et lesdits moyens d'acheminement d'air (21) comportant une zone résistive dite zone résistive de base (26).

15. Brûleur selon l'une des revendications précédentes, **caractérisé en ce que** lesdits moyens d'acheminement d'air (21) sont composés de ladite zone d'aspiration d'air (22), suivie d'une zone verticale de préchauffage de l'air (23) formant cheminée vers ladite zone de four (16).

16. Brûleur selon l'une des revendications précédentes, **caractérisé en ce que** ladite zone de préchauffage (23) comporte un élément résistif vertical sensiblement centré, dit résistance de préchauffage (24) et une zone résistive verticale dite zone résistive de parois de préchauffage (25) correspondant à une des parois verticales de ladite zone de préchauffage (23).

17. Brûleur selon la revendication 9, **caractérisé en ce que** ledit élément de protection (35) est en forme de S majuscule, selon une vue latérale.

18. Brûleur selon l'une des revendications précédentes, **caractérisé en ce que** la température dans ladite zone de four (16) varie entre 850 et 950°C et la température dans ladite zone de chauffage ouverte (20) est de l'ordre de 800°C.

## Claims

1. A burner enabling heating and high-temperature working of material such as glass, comprising electrical resistors (24, 27, 28, 29, 30), a heating device (2) formed of walls (12a, 12b, 12c, 12d), a housing (1) in which is fitted the heating device (2), and a furnace area (16) adapted to enable the high-temperature working of the said material, **characterized in that**:
the furnace area (16) has a U shape in the cross-sectional view, so that the upper branch (16b) and the lower branch (16a) are horizontal.

2. A burner according to the previous claim, **characterized in that** it comprises an open area for heating the said material (20) distinct from the furnace area (16).

3. A burner according to any of the previous claims, **characterized in that** it comprises a heat sink (31) adapted to be inserted in a volume (13) demarcated by the walls (12a, 12b, 12c, 12d) and the four electrical resistors (24, 28, 29, 30).

4. A burner according to claim 3, **characterized in that** the openings (8, 34) located on the housing (1) and the rear side (31c) of the heat sink (31) enable air convection.

5. A burner according to any of claims 3 to 4, **characterized in that** it comprises a first resistive area called the upper resistive area (17) corresponding to the said upper branch (16b) of the said U, and a second resistive area called the lower resistive area (19) corresponding to the said lower branch (16a) of the said U.

6. A burner according to claim 5, **characterized in that** the open heating area (20) is located above the upper resistive area (17) of the furnace.

7. A burner according to any of claims 3 to 6, **characterized in that** the metal heat sink (31) is made up of three sides (31 b, 31 c, 31d) that make up a U.

8. A burner according to any of claims 3 to 7, **characterized in that** the said heat sink (31) has on its parallel sides (31b, 31d) a cut-out (32b, 32d) shaped like a U.

9. A burner according to any of claims 3 to 8, **characterized in that** it comprises a protective element (35) adapted to fit in the said heat sink (31) in order to avoid direct contact between the said electric resistors (17, 18, 19, 25, 26) and the said material to work or the said element to heat.

10. A burner according to any of claims 3 to 9, **characterized in that** the heat sink (31) is formed so that the U-shaped openings (32b, 32d) of the sides (31b, 31d) match the U-shaped openings (16a, 16b) of the furnace area (16) and a U-shaped opening (5a, 5b) of the housing (1).

11. A burner according to any of the previous claims, **characterized in that** it comprises at least two electric resistive areas (17, 18, 19, 25) on two walls (16a, 16b, 16c) of the said furnace area (16).

12. A burner according to any of the previous claims, **characterized in that** the said furnace area (16) is demarcated by three electric resistive areas (17, 18, 19) forming the three walls (16a, 16b, 16c) of the said furnace area (16).

13. A burner according to any of the previous claims, **characterized in that** it comprises air circulation means (21) defining the inner volume (13) demarcated by the four walls in refractory material (12a, 12b, 12c, 12d) of the heating device (2).

14. A burner according to claim 13, **characterized in that** it comprises an air intake area (22), located at the connection between means for holding on the floor (11), on which the walls made of refractory material rest (12a, 12b, 12c, 12d), the said air intake area and the said air circulation means (21) comprising a resistive area called the base resistive area (26).

15. A burner according to any of the previous claims, **characterized in that** the said air circulation means (21) are comprised of the said air intake area (22), followed by a vertical air preheating area (23) forming a chimney to the said furnace area (16).

16. A burner according to any of the previous claims, **characterized in that** the said preheating area (23) comprises a vertical resistive element that is substantially centered, and called the preheating resistor (24) and a vertical resistive area called the preheating wall resistive area (25) which is one of the vertical walls of the said preheating area (23).

17. A burner according to claim 9, **characterized in that** the said protective element (35) is shaped like a capital S when viewed from the side.

18. A burner according to any of the previous claims, **characterized in that** the temperature in the said furnace area (16) varies from 850°C to 950°C and the temperature in the said open preheating area (20) is about 800°C.

## Patentansprüche

1. Brenner für das Erhitzen und Arbeiten bei hoher Temperatur mit einem Werkstoff wie z. B. Glas, mit elektrischen Widerständen (24, 27, 28, 29, 30), einer aus Wänden (12a, 12b, 12c, 12d) bestehenden Heizeinheit (2), einem Gehäuse (1), in das die Heizeinheit (2) passt und einem Ofenbereich (16) für das Arbeiten bei hoher Temperatur mit diesem Werkstoff, **dadurch gekennzeichnet dass**:
der Ofenbereich (16) im Querschnitt gesehen eine U-Form aufweist, sodass der obere Arm (16b) und der untere Arm (16a) waagerecht sind.

2. Brenner nach dem vorausgehenden Anspruch, **dadurch gekennzeichnet, dass** er einen offenen Heizbereich für den Werkstoff (20) umfasst, der sich vom Ofenbereich (16) unterscheidet.

3. Brenner nach einem der vorausgehenden Ansprüche, **dadurch gekennzeichnet, dass** er einen Wärmeableiter (31) umfasst, der in einen durch die Wände (12a, 12b, 12c, 12d) und die vier elektrischen Widerstände (24, 28, 29, 30) abgegrenzten Raum (13) eingefügt werden kann.

4. Brenner nach Anspruch 3, **dadurch gekennzeichnet, dass** die Öffnungen (8, 34) am Gehäuse (1) und auf der Rückseite (31c) des Wärmeableiters (31) eine Luftkühlung ermöglichen.

5. Brenner nach einem der Ansprüche 3 bis 4, **dadurch gekennzeichnet, dass** er einen ersten sogenannten oberen ohmschen Bereich (17) umfasst, der dem oberen Arm (16b) des U entspricht, sowie einen zweiten sogenannten unteren ohmschen Bereich (19), der dem unteren Arm (16a) des U entspricht.

6. Brenner nach Anspruch 5, **dadurch gekennzeichnet, dass** der offene Heizbereich (20) über dem oberen ohmschen Bereich (17) des Ofens liegt.

7. Brenner nach einem der Ansprüche 3 bis 6, **dadurch gekennzeichnet, dass** der metallische Wärmeableiter (31) aus drei Seiten (31 b, 31 c, 31 d) gebildet ist, die ein U ergeben.

8. Brenner nach einem der Ansprüche 3 bis 7, **dadurch gekennzeichnet, dass** der Wärmeableiter (31) an seinen parallelen Seiten (31 b, 31d) einen Einschnitt (32b, 32d) in U-Form aufweist.

9. Brenner nach einem der Ansprüche 3 bis 8, **dadurch gekennzeichnet, dass** er ein Schutzteil (35) umfasst, das in den Wärmeableiter (31) passt, wodurch ein direkter Kontakt der elektrischen Widerstände (17, 18, 19, 25, 26) mit dem zu bearbeitenden Werkstoff oder dem zu erhitzenden Element vermieden werden kann.

10. Brenner nach einem der Ansprüche 3 bis 9, **dadurch gekennzeichnet, dass** der Wärmeableiter (31) so geformt ist, dass die Öffnungen in U-Form (32b, 32d) der seitlichen Flanken (31 b, 31d) mit den Öffnungen in U-Form (16a, 16b) des Ofenbereichs (16) und einer Öffnung in U-Form (5a, 5b) des Gehäuses (1) übereinstimmen.

11. Brenner nach einem der vorausgehenden Ansprüche, **dadurch gekennzeichnet, dass** er mindestens zwei ohmsche Bereiche (17, 18, 19, 25) an zwei Wänden (16a, 16b, 16c) des Ofenbereichs (16) umfasst.

12. Brenner nach einem der vorausgehenden Ansprüche, **dadurch gekennzeichnet, dass** der Ofenbereich (16) durch drei ohmsche Bereiche (17, 18, 19) abgegrenzt ist, die die drei Wände (16a, 16b, 16c) des Ofenbereichs (16) bilden.

13. Brenner nach einem der vorausgehenden Ansprüche, **dadurch gekennzeichnet, dass** er Mittel zur Luftbeförderung (21) umfasst, die den durch die vier Wände aus einem feuerfesten Werkstoff (12a, 12b, 12c, 12d) der Heizeinheit (2) abgegrenzten Innenraum (13) definieren.

14. Brenner nach Anspruch 13, **dadurch gekennzeichnet, dass** er einen Luftansaugbereich (22) umfasst, der sich am Übergang zwischen den Mitteln zur Bodenhaltung (11) befindet, auf denen die Wände aus dem feuerfesten Werkstoff (12a, 12b, 12c, 12d) abgestützt sind, wobei der Luftansaugbereich und die Mittel zur Luftbeförderung (21) einen sogenannten ohmschen Grundbereich (26) umfassen.

15. Brenner nach einem der vorausgehenden Ansprüche, **dadurch gekennzeichnet, dass** die Mittel zur Luftbeförderung (21) aus dem Luftansaugbereich (22), gefolgt von einem senkrechten Bereich zum Vorwärmen der Luft (23), der einen Kamin zum Ofenbereich (16) bildet, bestehen.

16. Brenner nach einem der vorausgehenden Ansprüche, **dadurch gekennzeichnet, dass** der Vorwärmebereich (23) ein etwa mittiges senkrechtes ohmsches Element umfasst, das als Vorwärmwiderstand (24) bezeichnet wird, sowie einen senkrechten ohmschen Bereich, der als ohmscher Bereich von Vorwärmwänden (25) bezeichnet wird und einer der senkrechten Wände des Vorwärmbereichs (23) entspricht.

17. Brenner nach Anspruch 9, **dadurch gekennzeichnet, dass** das Schutzteil (35) seitlich gesehen die Form eines großen S hat.

18. Brenner nach einem der vorausgehenden Ansprüche, **dadurch gekennzeichnet, dass** die Temperatur im Ofenbereich (16) zwischen 850 und 950°C variiert und die Temperatur im offenen Heizbereich (20) ungefähr 800°C beträgt.
